# EUROPEAN PATENT SPECIFICATION

(11) **EP 0 372 352 B1**
(45) Date of publication and mention of the grant of the patent: **07.06.1995**
(21) Application number: 89121881.0
(22) Date of filing: 27.11.1989
(51) Int. Cl.: C12N 15/62, C07K 14/435, G01N 33/58, C12N 15/31, C12N 15/12

(54) **Aequorin fused with a protein having a specific-binding activity, its preparation, its purification and detection method by its use**
Mit einem Protein mit spezifischer Bindungsaktivität fusioniertes Aequorin, seine Herstellung, Reinigung und Nachweismethode
Aéquorine fusionnée avec une protéine ayant une activité de liaison spécifique, sa préparation, sa purification et méthode de détection l'utilisant

(30) Priority: 06.12.1988 JP 308424/88; 22.03.1989 JP 69862/89; 27.03.1989 JP 74742/89
(43) Date of publication of application: 13.06.1990
(73) Proprietor: Chisso Corporation, Osaka-shi Osaka-fu (JP)
(72) Inventor: Zenno, Shuhei, Yokohamashi Kanagawaken (JP); Inouye, Satoshi, San Diego CA 92122 (US)
(74) Representative: Kraus, Walter, Dr.

(56) References cited:
- EP-A- 0 157 235
- EP-A- 0 245 093
- EP-A- 0 302 381
- WO-A-84/04395
- WO-A-87/03304

## Description

This invention relates to aequorin fused with a protein having a specific-binding activity, a method for producing the same, a method for purifying the same and a detection method using the same.

Photoprotein aequorin is a calcium-binding protein isolated from photogenic Aequorea living in the ocean in the suberbs of Friday Harber Iseland, Washington State of U.S.A. Aequorin consists of a complex formed from apoaequorin as a protein part and coelenterazine as a substrate part by the medium of molecular form oxygen in the natural world, and it is characterized in that when calcium is bound to the above complex, it causes luminescence. Thus it is possible to measure calcium concentration utilizing the luminescence.

The present inventors have cloned the cDNA of apoaequorin from photogenic Aequorea by means of a recombinant DNA technique to determine the primary structure thereof (Japanese patent application laid-open No. Sho 61-135,586/1986). Next we have succeeded in intracellular and extracellular production of apoaequorin in Escherichia coli as a host using the above cDNA (Japanese patent application laid-open Nos. Sho 62-171,695/1987 and Sho 63-102,695/1988).

Further, we have prepared aequorin gene bound to a functional gene and succeeded in the production of a fused protein thereof (Japanese patent application Nos. Sho 62-196,031/1987 and Sho 63-308,424/1988). Furthermore we have developed a method for detecting metals making use of the luminescence of aequorin (Japanese patent application No. Sho 61-103,849/1986). The above invention is directed to a report having evidenced application to detection technique using an aequorin fused with a specific-binding protein.

Further, we have established a method for preparing a high purity, purified preparation of the fused protein in order to utilize it for an immunoassay (Japanese patent application No. Hei 1-69,862/1989).

However, detection technique for materials other than metals utilizing aequorin luminescence has not yet been reported; hence the present invention is directed to the first report having evidenced application to detection technique using aequorin bound to a substance having a specific-binding activity.

Now, the usefulness of aequorin may be anticipated by persons skilled in the art, and when aequorin is bound to a target substance by the medium of a specific-binding protein, it is possible to specifically detect the target substance by luminescence. The specific binding referred to herein means those utilizing antigen-antibody reaction, enzymatic reaction, specific binding to receptor, specific binding of nucleir acid to protein, etc. Further, aequorin fused with a protein having a specific-binding activity may be anticipated to be useful as an inspection medicine such as dignostic in view of the above-mentioned functions.

In view of the above-mentioned technical situation, the present inventors have made extensive research, and as a result, have succeeded in production of aequorin fused with a protein having an antibody-binding activity according to a recombinant DNA technique, purification into a high purity, of aequorin fused with a protein having an antibody-binding activity, produced according to a recombinant DNA technique; and development of a novel detection method by means of aequorin fused with a substance having a specific-binding activity.

EP-A-302 381 discloses a fused gene consisting of the gene coding for aequorin and the gene coding for a specific binding protein namely an enzyme, a DNA binding protein or an antibody. This document is cited under Article 54(3) EPC for the designated contracting states DE, FR and GB.

EP-A-254 093 relates to a method for the detection of metals by measuring the luminescence of a metal in the presence of aequorin, characterized in that the aequorin used has been reproduced by adding coelenterazine or an analogue thereof to an enzyme of aequorin (apoaequorin).

WO-A-87/03304 discloses a method for producing hybrid molecules useful in biological assays exhibiting luciferase activity and a second functionality comprising forming an association of three sequences of DNA by isolating a first sequence of DNA encoding luciferase activity, and associating with said first sequence of DNA a second sequence of DNA encoding said second functionality; and associating said first and second sequences of DNA with a third sequence of DNA encoding functions allowing for selecting, replicating, and expressing said hybrid molecules in a suitable host cell; and inserting said association of three sequences of DNA into said suitable host cell; and selecting and replicating said host cells; and isolating from said host cells said hybrid molecules.

As apparent from the above description, the object of the present invention is to provide aequorin fused with a protein having a specific-binding activity, and retaining aequorin activity, a substance having applied the same and a process having applied the same.

The present invention has the following constitutions (1) to (9):
(1) A method for producing a fusion gene of aequorin gene with a protein gene having a specific-binding activity, which method comprises cultivating Staphylococcus aureus or Escherichia coli having a plasmid having a replication-origin of Staphylococcus aureus or Escherichia coli having a fused gene of protein A gene with aequorin gene, introduced downstream from the promoter of gram-positive bacteria (Staphylococcus aureus) or gram-negative bacteria (Escherichia coli).
(2) A fusion gene of aequorin gene with a protein gene having a specific-binding activity produced according to the production method of item (1).
(3) A method for producing a fusion protein of aequorin with a protein having a specific-binding activity, which method comprises cultivating Staphylococcus aureus or Escherichia coli having a plasmid having a replication-origin of Staphylococcus aureus or Escherichia coli having a fused gene of protein A gene with aequorin gene, introduced downstream from the promoter of gram-positive bacteria (Staphylococcus aureus) or gram-negative bacteria (Escherichia coli).
(4) A fusion protein of aequorin with a protein having a specific-binding activity produced according to the production method of item (3).
(5) A method for detecting a target substance which comprises binding a substance having a specific-binding activity for said target substance to aequorin and binding the resulting bound substance to a target substance.
(6) A detection method according to item (5) wherein said bound substance is a substance having an aequorin activity and an antibody-binding activity.
(7) A detection method according to item (5) wherein said substance having a specific-binding activity is enzyme, antibody, protein A, protein G, DNA, RNA, DNA-binding protein or receptor.
(8) A detection method according to item (5) wherein said target substance is substrate, coenzyme, prosthetic group, antigen, antibody, DNA, RNA, hormone or transmitter.
(9) A method for detecting antibody which comprises binding protein A to aequorin and binding the resulting bound substance to antibody.

Figs. 1-10 each show a view illustrating the present invention.

Fig. 1 shows a flowsheet illustrating the construction steps of protein A-fused aequorin expression vectors pAAQ1 and pAAQ2.

Fig. 2 shows a nucleotide sequence in the vicinity of fusion of protein A gene with aequorin gene and an amino acid sequence corresponding thereto.

Fig. 3 shows the results of Western blotting wherein A refers to a case of using an antibody to aequorin and B refers to a case of using HRP-labeled IgG, and shows detection and identification of protein A-fused aequorin in pAAQ1 and pAAQ2.

Fig. 4 shows a flowsheet illustrating purification of aequorin-labeled protein A.

Fig. 5 shows the results of affinity (IgG Sepharose) chromatography.

Fig. 6 shows the results of gel filtration (Superose 12) chromatography.

Fig. 7 shows the results of reverse phase high performance liquid (Wakosil 5C4) chromatography.

Fig. 8 shows the effect of IgG concentration upon aequorin activity of aequorin-labeled protein A.

Fig. 9 shows the detection method of IgG using aequorin-labeled protein A, in a model manner.

Fig. 10 shows the detection sensitivity of immunoassay using aequorin-labeled protein A.

The constitutions and effectiveness of the present invention will be described in detail. The present invention is directed to aequorin fused with a protein having a specific-binding activity, and retaining aequorin activity, a production method of the same, and a detection method using the same. The aequorin is produced and the methods are carried out according to Examples mentioned later.

The present invention will be described referring to the accompanying drawings.

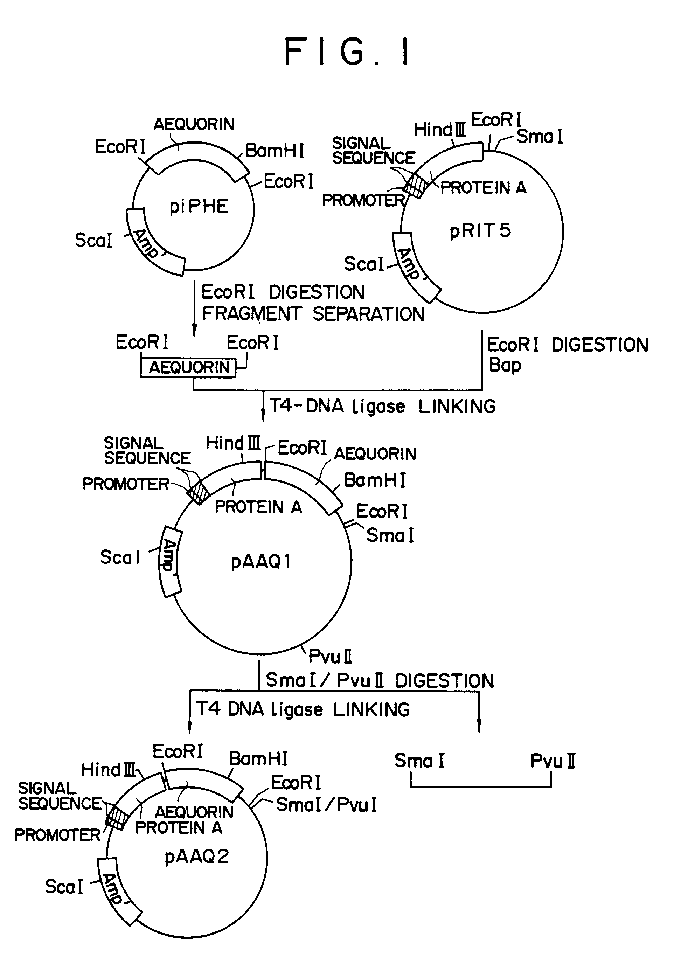

Fig. 1 shows the construction steps of the expression vector of aequorin fused with protein A.

Namely, a fragment of aequorin cDNA is separated from aequorin expression vector piP-HE (Japanese patent application laid-open No. Sho 63-102,695/1988) by EcoRI digestion, followed by inserting the fragment into EcoRI site of protein A-fused expression vector pRIT5 (made by Pharmacia Co., Ltd.) to prepare pAAQ1, subjecting pAAQ1 to Smal/Pvu II digestion and carrying out self-ligation to prepare pAAQ2. In order to indicate the direction of the substance to be constructed, promoter, signal sequence, gene resistant to Ampicillin and two or three restriction enzyme sites are indicated therein.

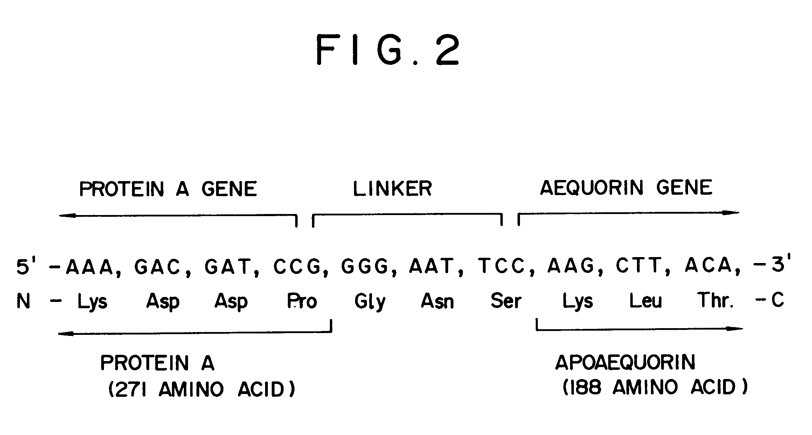

Fig. 2 shows a nucleotide sequence in the vicinity of fusion of protein A gene with aequorin gene and an amino acid sequence corresponding thereto.

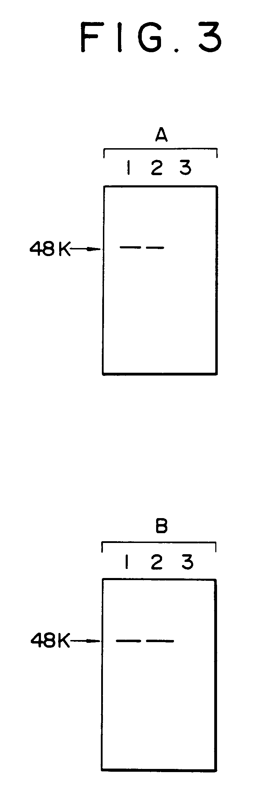

Fig. 3 shows the identification results of a protein A-fused aequorin according to Western blotting. A in the figure refers to a case using polyclonal antibody to aequorin and B refers to a case using a peroxidase-labeled antibody. Lane 1 refers to pAAQ1/JM83 strain, lane 2 refers to pAAQ2/JM83 strain and lane 3 refers to JM83 strain.

By using aequorin gene fused with a protein gene having a specific-binding activity obtained as described above, of the present invention (the first aspect of the invention), it has become possible to produce aequorin fused with a protein having a specific-binding activity, of the present invention (the second aspect of the invention).

The above production is relied on a known expression process except that the gene of the first aspect of the invention is used.

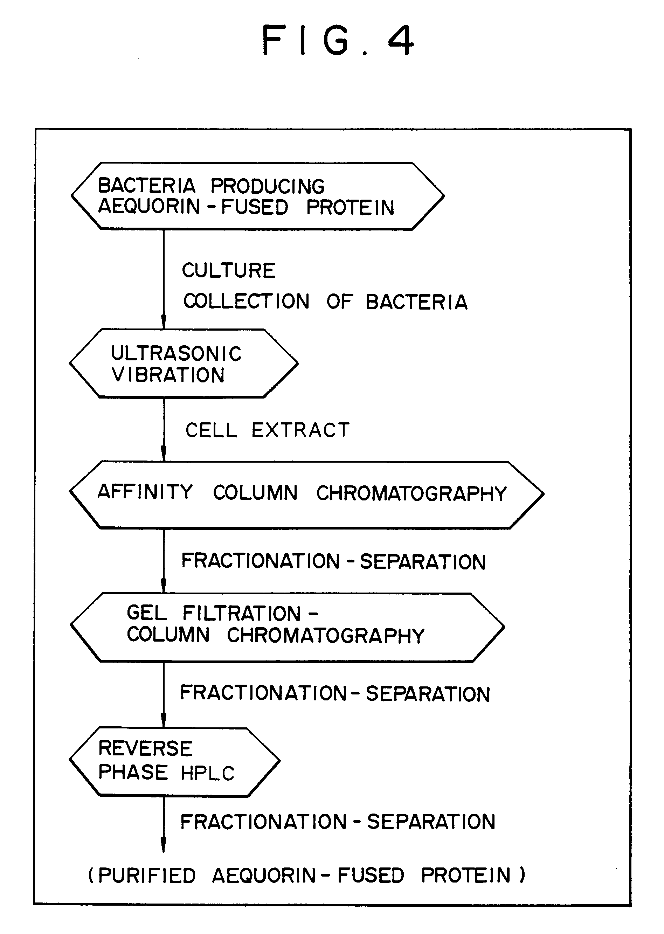

Fig. 4 shows a flowsheet illustrating purification of an aequorin-labeled, specifically bound protein. Aequorin-fused protein-producing bacteria are cultured under known conditions, followed by collecting the bacteria, subjecting the bacterial cells to ultrasonic vibration to prepare a cell extract and fractionating the cell extract according to affinity column chromatography to separate the fused protein. This operation utilizes a specific adsorption and is effective for enhancing the purity of the aimed material. Next, the above fraction is fractionated according to gel filtration column chromatography to separate the above fused protein depending on its size. This operation is effective for removing the fused protein involved in macromolecules such as membrane. Further, the aimed fraction is fractionated according to reverse phase HPLC to separate the above fused protein depending on hydrophobic properties. This operation is effective for preparing a target substance having a higher purity.

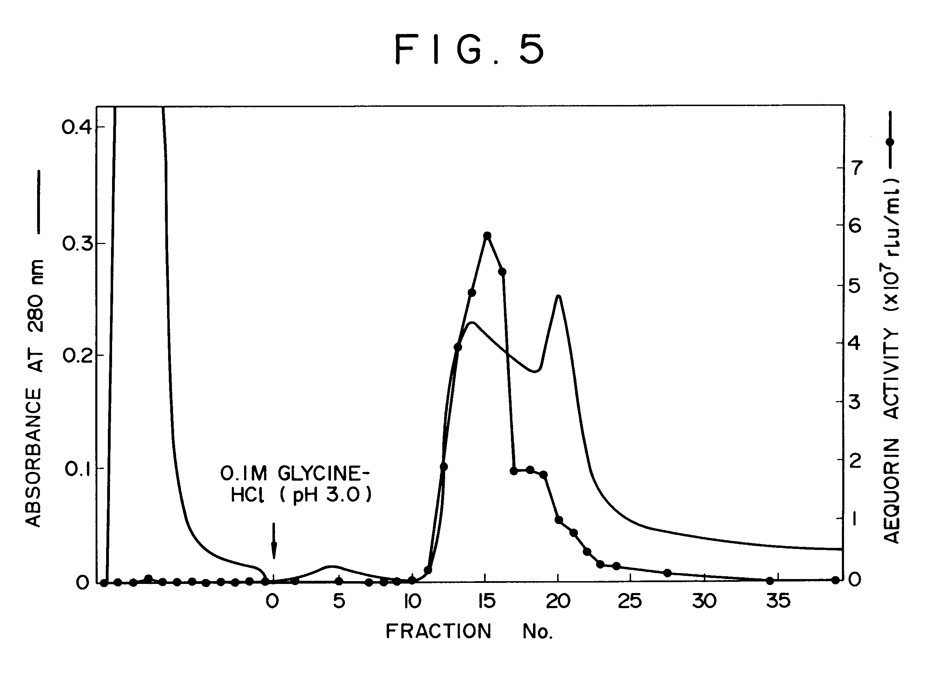

Fig. 5 shows the results of IgG Sepharose column chromatography (of sample of Example). In this figure, solid line refers to absorbance at 280 nm and black sphere refers to aequorin activity.

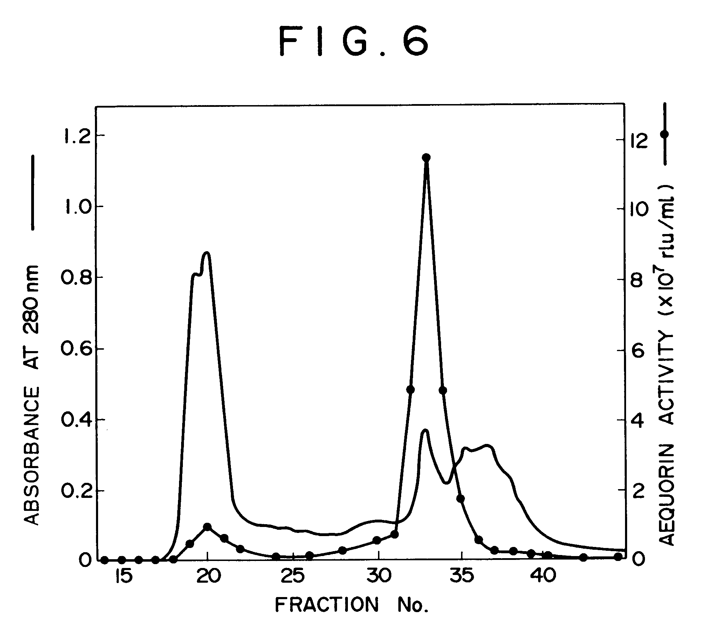

Fig. 6 shows the results of Superose 12 column chromatography. In this figure, solid line refers to absorbance at 280 nm and black sphere refers to aequorin activity.

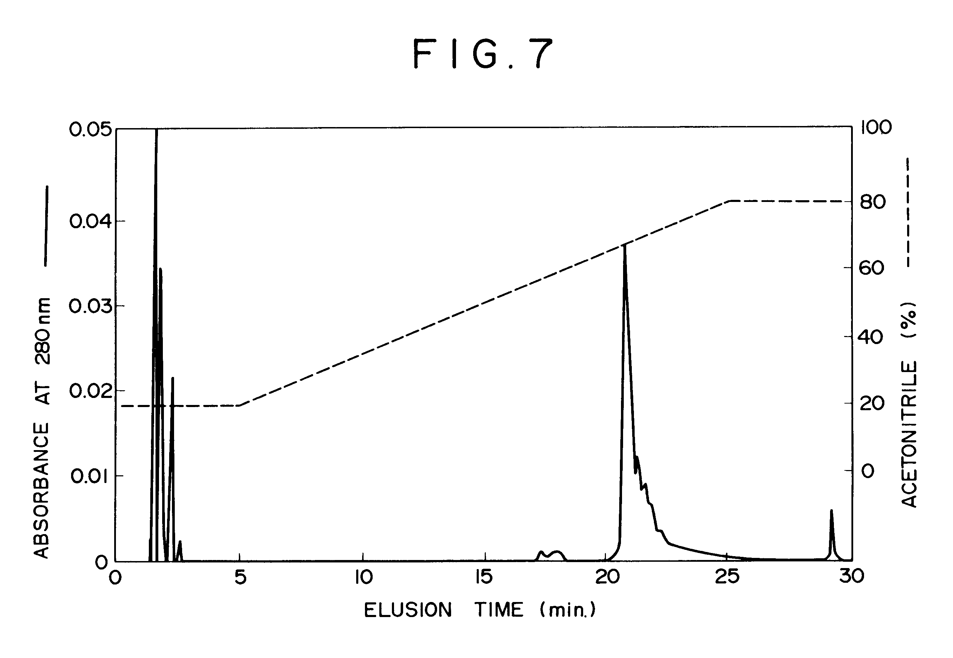

Fig. 7 shows the results of Wakosil 5C4 column chromatography. In this figure, solid line refers to absorbance at 280 nm and dotted line refers to proportion of acetonitrile.

When the highly purified, aequorin-labeled protein A is used for use applications such as inspection medicine, it is possible to directly detect antibody by means of luminescence and also it is possible to indirectly detect antigen. Namely, when an aequorin-labeled, specifically bound protein is used, it is possible to specifically detect various substances by luminescence, as a result of the above specific binding.

The detection method of the present invention will be described in detail. The present invention is directed to a detection method of a target substance using aequorin fused with a substance having a specific-binding activity, and retaining aequorin activity, and the detection method can be carried out for example according to a method illustrated in Examples mentioned later.

In the detection method of the present invention, a substance "having a specific-binding activity" referred therein means a substance to be specifically i.e. selectively bound with a target substance as mentioned below. For example, it means an antibody in the antigen-antibody reaction.

Further, the target substance referred to therein means a substance to be detected, as an object of the method of the present invention. For example, it means antigen in the antigen-antibody reaction.

Thus, the target substance and the substance having a specific-binding activity (hereinafter referred to often as specific-binding substance) have in some case a relative exchangeability. Such a specific-binding relationship between the target sugstance and the specific-binding substance is illustrated as follows:

**Table**

| Target substance | Substance having a specific-binding activity |
|---|---|
| Substrate | Enzyme |
| Coenzyme | Enzyme |
| Prosthetic group | Enzyme |
| Antigen | Antibody |
| Antibody | Protein A |
| Antibody | Protein G |
| DNA | DNA |
| DNA | RNA |
| RNA | DNA |
| RNA | RNA |
| DNA | DNA-binding protein |
| Hormone | Receptor |
| Transmitter | Receptor |

The binding of a substance having a specific-binding activity with aequorin has been as described above.

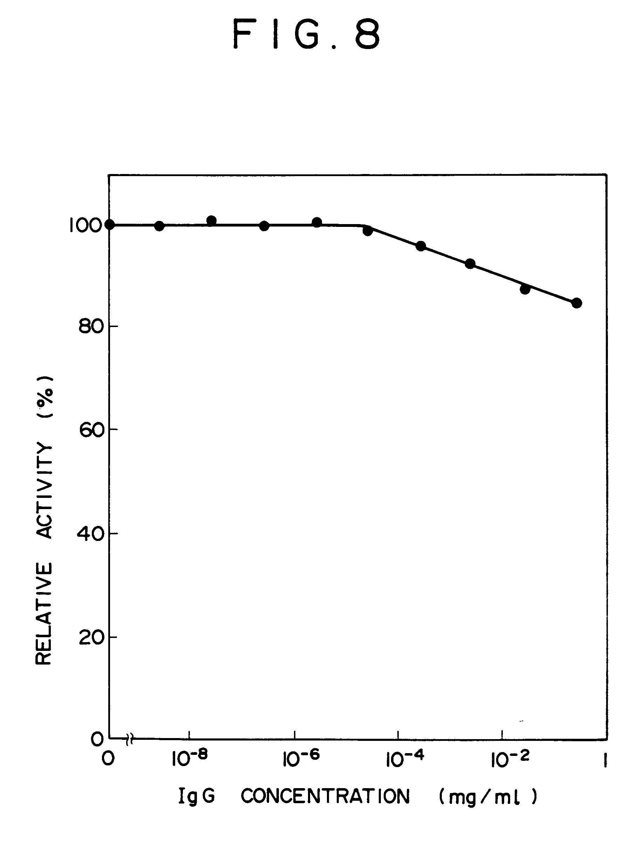

Fig. 8 shows an effect of IgG concentration upon the aequorin activity of aequorin-labeled protein A.

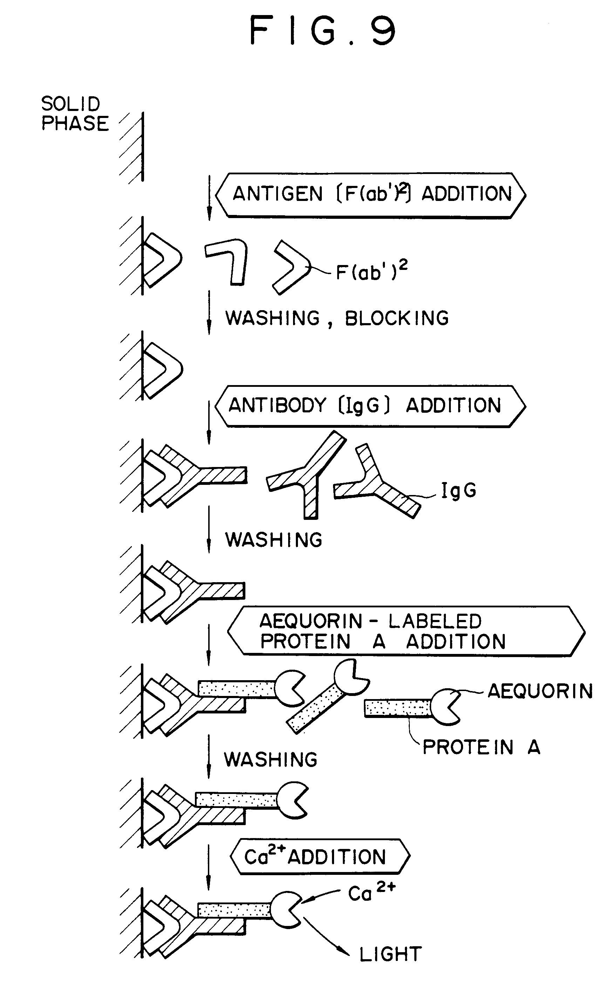

Fig. 9 shows a method for detecting an antibody using aequorin-labeled protein A in a model manner.

Namely, F(ab')² (antigen) containing no Fc fragment is adsorbed onto a solid phase such as polystyrene, followed by removing unadsorbed F(ab')², blocking the residue with a blocking agent (BSA), removing the blocking agent, contacting various concentrations of IgG (antibody), removing unadsorbed IgG, contacting IgG with an aequorin-labeled protein A, removing unadsorbed aequorin-labeled protein A, regenerating aequorin in the presence of coelenterazine and 2-mercaptoethanol, adding a large excess of Ca²⁺ to cause luminescence and determining IgG from the resulting luminescent quantity.

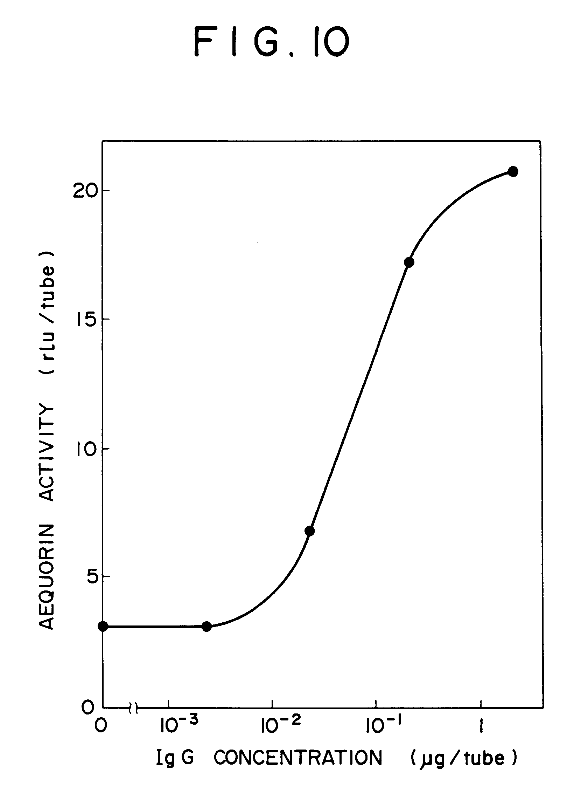

Fig. 10 shows the detection sensitivity of an immunoassay using aequorin-labeled protein A.

It is obvious to persons skilled in the art that an aequorin fused with a protein having a specific-binding activity and retaining aequorin activity, the production method of the same and the purification method of the same are useful. The specific-binding activity and luminescent activity of the above fused protein are applicable to a detection method relied on the presence or absence of luminescence. Further, since a suitable host such as Escherichia coli is employed, it is possible to produce the fusion protein in a large quantity.

According to the detection method of the present invention, a substance having a specific-binding activity to a target substance such as coenzyme, antigen, antibody, DNA, RNA, hormone, transmitter, etc. in the objective substances is chosen and labeled with aequorin, followed by binding the labeled substance with the above target substance and adding Ca²⁺ to make the aequorin luminescent; hence even when the target substance in the substances to be detected has an extremely low concentration, it is possible to detect the substance to be detected.

Further, since the substance to be labeled with aequorin is a substance having a specifc-binding activity to the target substance, the substance is not bound with substances other than the target substance in the substance to be detected so that the detection sensitivity is very high.

Furthermore, depending on the kind of the target substances, a substance having a specific-binding activity, corresponding thereto can be chosen; thus the method is applicable to a very broad range of objective substances (target substances), and hence its utilization range is very broad.

According to the above disclosure, it is possible for persons skilled in the art to practice the claimed invention. However, in order to more enhance understanding of this technique, procedures employed in the production of aequorin fused with protein A and having aequorin activity,its identification, its purification and a detection method using the same, each important for the present invention will be elucidated hereinafter.

### Example 1

### (Construction of protein A-fused aequorin expression vector)

Aequorin expression vector piP-HE (Japanese patent application laid-open No. Sho 63-102,695/1988) was digested with EcoRI and then treated at -80°C for 10 minutes.

The treated substance was subjected to agarose electrophoresis, followed by recovering aequorin cDNA-containing fragments into DEAE paper, twice washing the resulting DEAE paper with 0.1M NaCℓ, TE buffer solution (10 mM Tris hydrochloric acid, 1 mM EDTA, pH 8.0), thereafter 4 times eluting with 1M NaCℓ, TE buffer solution (pH 8.0), twice extracting the eluted DNA with phenol and precipitating with ethanol.

Protein A-fused expression vector pRIT5 (made by Pharmacia Co., Ltd.) was digested with EcoRI, followed by treating at -80°C for 10 minutes, further carrying out alkaline phosphatase treatment at 65°C for 3 hours, three times extracting with phenol and precipitating with ethanol.

The above-mentioned recovered aequorin cDNA fragment was combined with the protein A-fused expression vector each in a small quantity to link these by means of T4-DNA ligase.

A portion of the reaction liquor was transformed into Escherichia coli JM83, followed by spreading on a L plate and culturing at 37°C overnight.

The transformed strains were subjected to measurement of aequorin activity and chosen depending on the presence or absence of the activity. Further, with the transformed strains having the activity, a plasmid DNA was prepared and the size and the inserting direction of inserted DNA were confirmed by means of restriction enzyme digestion. This plasmid corresponds to protein A-fused aequorin expression vector pAAQ1 shown in Fig. 1.

pAAQ1 plasmid DNA was subjected to SmaI/Pvu II digestion, followed by treating at -80°C for 10 minutes, linking a portion of the reaction liquor with T4 DNA ligase, transforming the resulting substance, spreading the transformed substance on a L plate, culturing at 37°C overnight, preparing a plasmid DNA with the resulting transformed strain and confirming its size by means of restriction enzyme digestion.

The plasmid having a reduced size corresponds to protein A-fused aequorin expression vector pAAQ2 shown in Fig. 1, and actually forms a construction having deleted the shuttle vector portion between Escherichia coli and Staphylococcus aureus from pAAQ1.

As shown in Fig. 2, protein A-fused aequorin expression vectors pAAQ1 and pAAQ 2 express a fusion protein of signal peptide of protein A, protein A and apoaequorin, and the signal peptide of the resulting expression protein is cut when the expression protein passes through the inner membrane. Resultantly, a fusion protein of protein A and apoaequorin is accumulated in the periplasm region and it is considered that a protein A-fused aequorin of a molecular weight of 52,293 consisting of 462 amino acids, obtained by fusion of 271 amino acids originated from protein A and 188 amino acids originated from apoaequorin by the medium of Gly·Asn·Ser originated from a linker into the periplasm of Escherichia coli is exhibited.

Further, it is considered that when a transformed strain obtained by transforming pAAQ1 into Staphylococcus aureus belongting to Gram-positive bacteria is used, it is also possible to produce the above-mentioned protein A-fused aequorin (MW: 52,293) by secreting it into a medium.

Further, it is considered that the above fused protein may be easily purified at one step by making use of the specific binding of protein A to IgG and employing a IgG Sepharose column.

### Example 2

### (Preparation of plasmid DNA of protein A-fused aequorin expression vector)

A colony was planted on a 5 mℓ LB medium, followed by culture at 37°C overnight, transferring the resulting culture (1.5 mℓ) into an Eppendorf tube, subjecting it to centrifugal separation (12,000 rpm, 2 min.), removing the supernatant, suspending the resulting pellet in a glucose solution (60 »ℓ) (50 mM glucose, 25 mM Tris·hydrochloric acid (pH 8.0) and 10 mM EDTA), adding a 10 mg/mℓ lysozyme solution (40 »ℓ) prepared with a glucose solution just before its use), mildly mixing these, allowing the resulting substance to stand at room temperature for 5 minutes, adding a 0.2N NaOH, 1% SDS solution (200 »ℓ), mildly mixing these and allowing the resulting mixture to stand in ice for 5 minutes, adding a 5M potassium acetate solution (150 »ℓ), mildly mixing them, allowing to stand in ice for at least 5 minutes, subjecting the resulting material to centrifugal separation (12,000 rpm, 10 min., 4°C), transferring the supernatant into another Eppendorf tube, once extracting with phenol, precipitating with ethanol, subjecting to centrifugal separation (12,000 rpm, 5 min.), washing the resulting pellet with 70% ethanol, drying in vacuo, dissolving the pellet in a TE buffer (pH 8.0) (50 »ℓ), adding a RNase A (0.5 mg/mℓ) solution (1 »ℓ) so as to give a concentration of 10 »g/mg, keeping the mixture at 37°C for 30 minutes, adding 20% polyethylene glycol (PEG) 6000/2.5 M NaCℓ (30 »ℓ), sufficiently mixing the mixture, allowing to stand in ice for at least one hour, subjecting to centrifugal separation (1200 rpm, 5 min.), removing the supernatant, once washing the resulting pellet with 70% ethanol, drying in vacuo, and dissolving the pellet in a suitable quantity of TE (pH 8.0).

### Example 3

### (Measurement of aequorin activity)

A 50% glycerol stock (50 uℓ)obtained from culture of a transformed strain was planted on a LB medium (10 mℓ), followed by culturing at 37°C overnight, transferring the resulting culture (1.5 mℓ) into an Eppendorf tube, subjecting it to centrifugal separation (12,000 rpm, 2 min.), removing the supernatant, suspending the resulting pellet in a buffer solution of 30 mM Tris·HCℓ (pH 7.6) and 10 mM EDTA (pH 7.6), subjecting the cells to an ultrasonic vibration, further subjecting the resulting material to centrifugal separation (12,000 rpm, 5min.) and measuring the aequorin activity in the supernatant. The reaction solution (200 »ℓ) contains 30 mM Tris·HCℓ (pH 7.6), 10 mM EDTA (pH 7.6) buffer solution, 1 mg/mℓ coelenterazine (1 »ℓ), 2-mercaptoethanol (4 »ℓ) and a raw enzyme extraction solution.

The reaction solution was allowed to stand at 4°C overnight, followed by transferring a portion of the reaction solution into a cuvette of lumino-photometer (TD-4000, tradename of Laboscience Co., Ltd.), pouring 30 mM CaCℓ₂ (100 »ℓ) therein and measuring its luminescent activity.

Aequorin activities originated from plasmids pAAQ1 and pAAQ2 were detected. The activity quantities were 9.3 × 10⁵ r.l.u./mℓ culture in the case of pAAQ1 and 2.0 × 10⁵ r.l.u./mℓ culture in the case of pAAQ2. This shows that aequorin having protein A fused on the N-terminus side is produced and also that the fusion protein has an aequorin activity.

### Example 4

### (Identification of protein A-fused aequorin according to Western blotting)

The overnight cultures of pAAQ1/JM83 strain and pAAQ2/JM83 were separated by subjecting them to SDS polyacrylamide electrophoresis according to Laemmli method (Laemmli, U.K. (1970) Nature 277, 680). The resulting gel was subjected to Western blotting according to Towbin et al's method (Towbin, H., Staehlin, T. and Gordon, J., Proc. Natl. Acad. Sci., USA, 76 4350 (1979)).

Namely, a band on the gel was transferred onto nitrocellulose by means of an electroblotter, followed by reacting the nitrocellulose filter with an antibody and further subjecting 4-chloro-1-naphthol to color development by means of a peroxydase.

As the antibody, rabbit IgG (polyclonal antibody) to a recombinant aequorin prepared in a conventional manner and Horseradish peroxydase (HRP)-labeled goat anti-rabbit IgG were used.

As a kit of a color development-detecting system using the HRP-labeled antibody (immune blot), a commercially available product (purchased from BIO-RAD Co., Ltd.) was used.

Fig. 3 shows the results of Western blotting, and A shows the case of use of aequorin antibody and B shows the case of use of HRP-labeled IgG. Both the cases exhibited bands of about 48 killodaltons in pAAQ1 (lane 1) and pAAQ2 (lane 2). Further, in the case of JM83 strain (lane 3) as a control, no significant band was detected.

This fact evidences that the bands of about 48K originated from pAAQ1 and pAAQ2 contain apoaequorin and protein A, and further evidences that the bands have an antibody-binding activity of protein A. The size of this 48K was somewhat less than the size of the expected fused protein of 52K, but it was an approximate value thereto.

In summary of the above results, it is presumed that the fusion protein of protein A and apoaequorin of about 48K expressed by pAAQ1 and pAAQ2 retains both the activities (antibody-binding activity and luminescent activity) and use of the present fusion protein makes it possible to detect antibodies by means of luminescence.

### Example 5

### (Preparation of the cell extract of aequorin-labeled protein A)

A glycerol stock at -20°C of aequorin-labeled protein A-producing bacteria (pAAQ1/JM 83 strains, Japanese patent application No. Sho 63-308424/1988) (20 »ℓ) was inoculated in L-broth (1% tripton, 0.5% yeast extract and 0.5% NaCℓ) (10 m»), followed by adding 200 mg/mℓ ampicillin (10 »ℓ), subjecting the mixtuure to shaking culture at 37°C overnight, transferring the resulting culture into a centrifugal tube, subjecting it to centrifugal separation at 10,000 rpm, at 4°C for 2 minutes to collect precipitates (bacterial bodies), suspending them in a buffer of 30 mM Tris HCℓ (pH 7.6) and 10 mM EDTA, of ¹/₁₀ volume of the precipitates, subjecting the bacterial bodies to ultrasonic vibration, further subjecting the resulting material to centrifugal separation at 15,000 rpm, at 4°C for 2 minutes to obtain a supernatant which was regarded as the cell extract of an aequorin-labeled protein A, and measuring the aequorin activity and protein quantity thereof. As a result, the aequorin activity was 12.7 × 10⁷ r.l.u./mℓ, the protein quantity was 14.4 mg/mℓ and the specific activity was 8.82 × 10⁶ r.l.u./mg.

### Example 6

### (Preparation of affinity-purified, aequorin-labeled protein A)

The cell extract of aequorin-labeled protein A (20 mℓ) was subjected to affinity chromatography with IgG Sepharose 6FF (1.5φ × 4.5 cm, made by Pharmacia Co., Ltd.), followed by successively washing with 50 mM Tris HCℓ (pH 7.6), 150 mM NaCℓ, 0.05% Tween 20 buffer (100 mℓ) and then with 5 mM ammonium acetate (pH 5.0) buffer (20 mℓ). The respective flow rates of adsorption and washing were 2 mℓ/min., and fractions were separated each in 10 mℓ. The elution was carried out with 0.1 M glycine HCℓ (pH 3.0) buffer. The flow rate was 0.2 mℓ/min. and fractions were separated each in 1 mℓ.

The peak after the elution was regarded as affinity-purified, aequorin-labeled protein A. The aequorin activity and the protein quantity were measured. After the elution, the 15th fraction had a highest specific activity, 1.11 × 10⁸ r.l.u./mg. With the 11th to 39th fractions, the percentage recovery of aequorin activity was 12.2%. Thus, in the case of No. 15 fraction, the specific activity increased by 12.6 times as compared with the cell extract.

As shown in Fig. 5, the present operation is effective for separating and purifying the objective substance from a roughly purified target substance. With regard to affinity chromatography utilizing a specific binding other than that between antibody and protein A, too, the method is, of course, also useful.

### Example 7

### (Preparation of gel filtration-purified, aequorin-labeled protein A)

An affinity-purified, aequorin-labeled protein A was adjusted so as to have a pH of 7 to 8 with 27% aqueous ammonia, followed by concentrating the substance by means of a concentrator (Centricon 30, tradename of Grace Japan Co., Ltd.), subjecting the concentrated, affinity-purified, aequorin-labeled protein A (50 »ℓ) to gel filtration chromatography by means of Superose 12 (1φ × 30 cm, tradename of Pharmacia Co., Ltd.), and separating the resulting fractions each in a quantity of 0.4 mℓ, at a flow rate of 0.2 mℓ/min., using a buffer of 50 mM sodium phosphate (pH 7.6) and 150 mM NaCℓ. Fractions active in terms of aequorin activity were regarded as gel filtration-purified, aequorin-labeled protein A.

With various samples, aequorin activity and protein quantity were measured. As a result, the aequorin activity of the affinity-purified, concentrated target substance was 21.4 × 10⁸ r.l.u./mℓ, its protein quantity was 66.0 mg/mℓ and its specific activity was 3.24 × 10⁷ r.l.u./mg. The 33rd fraction had a highest activity, 2.53 × 10⁸ r.l.u./mg. The specific activity rose to 7.8 times the one prior to fractionation by means of gel filtration.

With the 31st to 36th fractions, the percentage recovery of aequorin activity was 90.9%.

As shown in Fig. 6, the present operation is effective for removing the decomposition substance of the objective product and substances contained therein, present in admixture with the affinity-purified target substance.

### Example 8

### (Preparation of HPLC-purified, aequorin-labeled protein A)

A gel filtration-purified, aequorin-labeled protein A (fraction No. 33) (100 »ℓ) was subjected to reverse phase HPLC by means of Wakosil 5C4 (4.6φ × 100mm), using 0.1% trifluoroacetic acid, water/acetonitrile system as solvent and at a flow rate of 0.8 mℓ/min., to separate main peak portions, concentrating and measuring aequorin activity. As a result, the percentage recovery of aequorin activity was 21.6%.

As shown in Fig. 7, the present operation was effective for removing impurities which could not be separated by means of gel filtration operation.

### Example 9

### (Determination of protein)

The determination of the protein was carried out according to a dyestuff-binding method using Coomasie Brilliant Blue (Bradford, M,M. (1976), Anal. Biochemy 72, 248). Namely, the determination was carried out using a commercially available protein assay kit I. A dyeing solution (0.6 mℓ) was added to the sample (2.4 mℓ) suitably diluted, followed by mixing and measuring the absorbance at 595 nm after 15 minutes. As a standard, cow. r globuline was used.

### Example 10

### (Effect of IgG concentration upon aequorin activity of aequorin-labeled protein A)

Gel filtration-purified, aequorin-labeled protein A (9.6 »g/mℓ) (10 »ℓ), goat IgG (various concentrations) (10 »ℓ), 2-mercaptoethanol (1 »ℓ), coelenterazine (2 mg/mℓ) (1 »ℓ), 30 mM Tris-HCℓ and 10 mM EDTA (pH 7.6) buffer (78 »ℓ), the total volume being 100 »ℓ, were allowed to stand at 4°C for 15 hours to regenerate aequorin.

A portion of the reaction solution was transferred into a cuvette of a lumiphotometer (TD-4000, tradename of Laboscience Co., Ltd.), followed by pouring 30 mM CaCℓ₂ (100 »ℓ) therein to measure its luminescent quantity.

Fig. 8 shows the summerized results. Although the aequorin activity gradually lowered after the vicinity of an IgG concentration of 10⁻⁴ mg/mℓ, 85% of the activity was retained even at an IgG concentration of 0.28 mg/mℓ. This evidences that the aequorin activity is sufficient even in a state where aequorin-labeled protein A is bound to IgG or at a state where they are coexistent, and also strongly suggests that its application to a method of measuring enzyme immunity is possible.

### Example 11

### (Detection of IgG by means of aequorin-labeled protein A based on sandwich method)

A polystyrene tube (10φ × 65 mm) was immersed in 2N NaOH at 60°C for 30 minutes, followed by washing, washing off NaOH with water and drying at 50°C. As shown in Fig. 9, 26 »g/mℓ rabbit F(ab')² (100 »ℓ) was added into the NaOH-treated polystyrene tube so that it might not be attached onto the lateral wall of the tube, followed by incubation at 37°C for 3 hours.

After rabbit F(ab')² was removed, a blocking buffer (1.0% Bovine serum albumin in 20 mM Tris-HCℓ, pH 7.5, and 5,500 mM NaCℓ [TBS]) (2.5 mℓ) was added, followed by incubation at 37°C for 2.5 hours.

After the blocking buffer was removed, washing was 10 times carried out with 20 mM Tris-HCℓ, pH 7.5, 500 mM NaCℓ and 0.05% Tween-20 [TTBS] (2.5 mℓ).

After TTBS was completely removed by a centrifuge, various concentrations (2.5 »g/mℓ to 250 »g/mℓ) of goat IgG (anti-rabbit F(ab')²) (100 »ℓ) were added, followed by incubation at 37°C for 1.5 hour. After the goat IgG was removed, 96 »g/mℓ aequorin-labeled Protein A (gel filtration-purified sample) (100 »ℓ) was added, followed by incubation at 37°C for 1.5 hour. After the aequorin-labeled protein A was removed, washing was ten times carried out with TTBS (2.5 mℓ).

After TTBS was completely removed by a centrifuge, 10 »g/mℓ coelenterazine, 0.5% 2-mercaptoethanol in 30 mM Tris-HCℓ (pH 7.6) and 10 mM EDTA (100 »ℓ) were added, followed by allowing the mixture to stand at 4°C for 15 hours.

The resulting material was transferred into the tube of a lumiphotometer (TD-4000, tradename of Laboscience Co., Ltd.), followed by pouring 300 mM CaCℓ₂ (100 »ℓ) therein and measuring its luminescent quantity. The results are shown in Fig. 10. A proportional relationship of the quantity of antibody (IgG) to the luminescent quantity is observed in the range of 10⁻² to 1 »g/tube, and it is seen that determination of the antbody is possible in the range.

The present detection process is applicable to a broad range of uses by making use of various specific bindings. Further, aequorin-labeled, specifically bound substances can be easily prepared by employing binding methods such as maleimide method, glutaraldehyde method, etc. Further, accompanying improvements in substrates (luminescent bodies) or luminescence-detectors, it is experted that a detection system having a higher sensitivity will be achieved.

## Claims

1. A method for producing a fusion gene of aequorin gene with a protein gene having a specific-binding activity, which method comprises cultivating Staphylococcus aureus or Escherichia coli having a plasmid having a replication-origin of Staphylococcus aureus or Escherichia coli having a fused gene of protein A gene with aequorin gene, introduced downstream from the promoter of gram-positive bacteria (Staphylococcus aureus) or gram-negative bacteria (Escherichia coli).

2. A fusion gene of aequorin gene with a protein gene having a specific-binding activity produced according to the production method of claim 1.

3. A method for producing a fusion protein of aequorin with a protein having a specific-binding activity, which method comprises cultivating Staphylococcus aureus or Escherichia coli having a plasmid having a replication-origin of Staphylococcus aureus or Escherichia coli having a fused gene of protein A gene with aequorin gene, introduced downstream from the promoter of gram-positive bacteria (Staphylococcus aureus) or gram-negative bacteria (Escherichia coli).

4. A fusion protein of aequorin with a protein having a specific-binding activity produced according to the production method of claim 3.

5. A method for detecting a target substance which comprises binding a substance having a specific-binding activity for said target substance to aequorin and binding the resulting bound substance to a target substance.

6. A detection method according to claim 5 wherein said bound substance is a substance having an aequorin activity and an antibody-binding activity.

7. A detection method according to claim 5 wherein said substance having a specific-binding activity is enzyme, antibody, protein A, protein G, DNA, RNA, DNA-binding protein or receptor.

8. A detection method according to claim 5 wherein said target substance is substrate, coenzyme, prosthetic group, antigen, antibody, DNA, RNA, hormone or transmitter.

9. A method for detecting antibody which comprises binding protein A to aequorin and binding the resulting bound substance to antibody.

## Patentansprüche

1. Verfahren zur Herstellung eines Fusions-Gens aus einem Äquorin-Gen und einem Protein-Gen, das eine spezifische Bindungsaktivität aufweist, umfassend das Züchten von Staphylococcus aureus oder Escherichia coli, die ein Plasmid aufweisen, das einen Replikations-Origin von Staphylococcus aureus oder Escherichia coli aufweist, das ein fusioniertes Gen aus Protein-A-Gen und Äquorin-Gen aufweist, das stromabwärts vom Promotor der grampositiven Bakterien (Staphylococcus aureus) oder der gramnegativen Bakterien (Escherichia coli) eingeführt worden ist.

2. Fusions-Gen aus einem Äquorin-Gen und einem Protein-Gen, das eine spezifische Bindungsaktivität aufweist, hergestellt nach dem Herstellungsverfahren gemäß Anspruch 1.

3. Verfahren zur Herstellung eines Fusionsproteins aus Äquorin und einem Protein, das eine spezifische Bindungsaktivität aufweist, umfassend das Züchten von Staphylococcus aureus oder Escherichia coli, die ein Plasmid aufweisen, das einen Replikations-Origin von Staphylococcus aureus oder Escherichia coli aufweist, das ein fusioniertes Gen aus Protein-A-Gen und Äquorin-Gen aufweist, das stromabwärts vom Promotor der grampositiven Bakterien (Staphylococcus aureus) oder der gramnegativen Bakterien (Escherichia coli) eingeführt worden ist.

4. Fusionsprotein aus Äquorin und einem Protein, das eine spezifische Bindungsaktivität aufweist, hergestellt nach dem Herstellungsverfahren gemäß Anspruch 3.

5. Verfahren zur Detektion einer Zielsubstanz, umfassend das Binden einer Substanz mit einer spezifischen Bindungsaktivität für die Zielsubstanz an Äquorin und das Binden der resultierenden gebundenen Substanz an eine Zielsubstanz.

6. Detektionsverfahren nach Anspruch 5, dadurch **gekennzeichnet,** daß die genannte gebundene Substanz eine Substanz ist, die eine Äquorinaktivität und eine antikörperbindende Aktivität aufweist.

7. Detektionsverfahren nach Anspruch 5, dadurch **gekennzeichnet,** daß die Substanz, die eine spezifische Bindungsaktivität aufweist, ein Enzym, Antikörper, Protein A, Protein G, DNA, RNA, DNA-bindendes Protein oder ein Rezeptor ist.

8. Detektionsverfahren nach Anspruch 5, dadurch **gekennzeichnet,** daß die Zielsubstanz ein Substrat, ein Coenzym, eine prosthetische Gruppe, ein Antigen, ein Antikörper, DNA, RNA, ein Hormon oder ein Transmitter ist.

9. Verfahren zur Antikörperdetektion, umfassend das Binden von Protein A an Äquorin und das Binden der resultierenden gebundenen Substanz an einen Antikörper.

## Revendications

1. Procédé pour la production d'un gène fusionné de gène d'aéquorine avec un gène de protéine ayant une activité de liaison spécifique, qui comprend la culture de *Staphylococcus aureus* ou d'*Escherichia coli* ayant un plasmide ayant une origine de réplication de *Staphylococcus aureus* ou d'*Escherichia coli* ayant un gène fusionné d'un gène de protéine A avec un gène d'aéquorine, introduit en aval du promoteur de bactéries gram-positives (*Staphylococcus aureus*) ou de bactéries gram-négatives (*Escherichia coli*).

2. Gène fusionné de gène d'aéquorine avec un gène de protéine ayant une activité de liaison spécifique produit suivant le procédé de la revendication 1.

3. Procédé pour la production d'une protéine fusionnée d'aéquorine avec une protéine ayant une activité de liaison spécifique, qui comprend la culture de *Staphylococcus aureus* ou d'*Escherichia coli* ayant un plasmide ayant une origine de réplication de *Staphylococcus aureus* ou d'*Escherichia coli* ayant un gène fusionné d'un gène de protéine A avec un gène d'aéquorine, introduit en aval du promoteur de bactéries gram-positives (*Staphylococcus aureus*) ou de bactéries gram-négatives (*Escherichia coli*).

4. Protéine fusionnée d'aéquorine avec une protéine ayant une activité de liaison spécifique produite suivant le procédé de la revendication 3.

5. Procédé pour la détection d'une substance cible, qui comprend la liaison d'une substance ayant une activité de liaison spécifique pour cette substance cible à l'aéquorine et la liaison de la substance liée résultante à une substance cible.

6. Procédé de détection suivant la revendication 5, dans lequel cette substance liée est une substance ayant une activité aéquorine et une activité de liaison d'anticorps.

7. Procédé de détection suivant la revendication 5, dans lequel cette substance ayant une activité de liaison spécifique est une enzyme, un anticorps, une protéine A, une protéine G, un ADN, un ARN, un récepteur ou une protéine se liant à un ADN.

8. Procédé de détection suivant la revendication 5, dans lequel cette substance cible est un substrat, une coenzyme, un groupe prosthétique, un antigène, un anticorps, un ADN, un ARN, une hormone ou un transmetteur.

9. Procédé pour la détection d'un anticorps qui comprend la liaison de protéine A à l'aéquorine et la liaison de la substance liée résultante à l'anticorps.
